Europäisches Patentamt

European Patent Office

Office européen des brevets

(11) Publication number:

**0 050 654**

**B1**

(12) **EUROPEAN PATENT SPECIFICATION**

(45) Date of publication of patent specification: **18.12.85**

(21) Application number: **81901257.6**

(22) Date of filing: **22.04.81**

(86) International application number:
**PCT/US81/00532**

(87) International publication number:
**WO 81/02982 29.10.81 Gazette 81/25**

(51) Int. Cl.⁴: **A 61 M 11/00**

(54) **INHALATION SPIROMETRIC DEVICE FOR ADMINISTERING PULMONARY MEDICATION.**

(30) Priority: **22.04.80 US 142697**

(43) Date of publication of application:
**05.05.82 Bulletin 82/18**

(45) Publication of the grant of the patent:
**18.12.85 Bulletin 85/51**

(84) Designated Contracting States:
**AT CH DE FR GB LI NL SE**

(56) References cited:
**DE-A-2 803 993**
**FR-A- 493 816**
**FR-A- 773 018**
**US-A- 844 449**
**US-A-1 781 735**
**US-A-1 853 242**
**US-A-2 007 330**
**US-A-3 455 294**
**US-A-3 473 529**
**US-A-3 507 278**
**US-A-3 635 214**
**US-A-3 861 396**

(73) Proprietor: **Key Pharmaceuticals, Inc.**
**50 N.W. 176th Street**
**Miami, Florida 33169 (US)**

(72) Inventor: **SACKNER, Marvin A.**
**300 W.Rivo Alto Drive**
**Miami Beach, FL 33139 (US)**
Inventor: **WATSON, Herman**
**10841 S.W. 156th Street**
**Miami, FL 33157 (US)**

(74) Representative: **Cheyne, John Robert Alexander Mackenzie et al**
**HASELTINE LAKE & CO. 28 Southampton Buildings Chancery Lane**
**London WC2A 1AT (GB)**

(56) References cited:
**US-A-4 119 097**
**US-A-4 226 233**
**US-A-4 241 740**

Courier Press, Leamington Spa, England.

## Description

The present invention relates to inhalation apparatus and methods suitable for administering pulmonary medication. Further, the apparatus and methods are adapted to permit incentive spirometry with use of the inhalation apparatus.

Apparatus for administering drugs to pulmonary tissue are known in the prior art. Conventional apparatus for administering such drugs are shown, for instance, in U.S. Patent No. 4,174,712 and other references known to those skilled in the art. These apparatuses have in common a pressurized inhaler bottle containing aerosol propellent and a medication to be applied to the pulmonary tissue. A nebulizer is associated with the pressurized inhaler bottle whereby the actuation of the combination nebulizer-pressurized inhaler bottle introduces a predetermined dosage of medication to the mouth and oral cavity area of the patient.

Difficulties with administering this type of medication have been experienced in using the prior art devices. For instances, it is found that direct spraying of the medicant into the oral cavity leaves a substantial portion of the dosage on the patient's tongue and otherwise not in the pulmonary tissue where the drug is required. This particularly poses a problem for steroidal inhalants which have strict limits on total dosage, and on side effects such as promoting fungile growth on the tongue. Further, some patients have difficulty in effecting the inhalation of the medication. The resulting process of delivering the drugs to the pulmonary tissue results in only a portion of the required dosage reaching the tissue area. This requires repeated dosages to be given to the patient so that sufficient medication reaches the pulmonary tissue. In addition a portion of the inhaled medication is not absorbed, thus it is lost upon expiration. Repeating the dosage also places an unnecessary amount of propellant contained in the pressurized aerosol mist into the respiratory system than is otherwise desired. The processes and apparatus of the prior art therefore do not provide a uniform distribution of pulmonary medication in the respiratory tracts. A further difficulty resulting from these prior techniques is that the rate of distribution of the pulmonary medication is not constant from patient to patient or dosage to dosage.

Conventional inhalers require a simultaneous actuation of a nebulizer and aerosol container with the act of inhalation. This degree of coordination required for use of these devices makes it difficult to administer pulmonary medication to young children and patients unfamiliar with the technique. Further, it has been found that a long and slow inspiration period promotes an efficient distribution of medication to partially occluded airways. The conventional inhalers are not adapted to a long and slow inspiration period as actuation and inhalation steps are conducted simultaneously, often creating a bolus of concentrated medication in the inhaled air.

The prior art incentive spirometer has as a rule been a complicated mechanism offering some difficulty to patients to operate. Further, spirometer technology has been directed to volumetric measurements of the inspiration of a patient, although incentive spirometers have been used for conducting therapeutic and prophylactic respiratory maneuvers. These devices, however, do not provide during use a negative thoracic pressure for inhibiting a rapid inhalation. By inhibiting the rapid inhalation, certain therapeutic benefits are realised in an incentive spirometer not attainable in these prior art devices.

As in the case in the inhalation devices, incentive spirometers are desired which encourage a long and slow inspiration period. During respiratory maneuvers, it is advantageous to provide a visual or audible means for a patient to gauge the breathing progress. Some prior art devices are flow controlled, whereby the flow of air is monitored by the device. These devices, however, encourage a rapid inspiratory rate rather than a long, slow inspiration period. Other apparatus detect the volume of air respiration achieved and are in many instances cumbersome and complicated. Typically, they may comprise a calibrated bellows on a flat surface such as a bedside table and are interfaced to the patient with a long flexible hose. These devices have as a final goal a certain volume inspired. There is no direct feedback to the patient as to his progress during breathing until the final volume is achieved.

Furthermore, most of the prior art devices are not capable of providing a dosage of medication while conducting therapeutic maneuvers. The combination of an incentive spirometer with a medicinal inhaler is most promising in treating postoperative atelectasis and for clearing the small airways of the lungs.

FR—A—773018 discloses an inhalation device comprising a mouthpiece having one end for communication with an oral cavity, the mouthpiece communicating with a through channel which has an inlet for receiving a gas or vapour and an outlet directed away from the said one end of the mouthpiece, the outlet of the through channel communicating with an open end of an inflatable envelope having surfaces for confining the gas or vapour, the device further comprising means for supplying a predetermined amount of gas or vapour to the inlet so that, in use, a quantity of gas or vapour enters the envelope and is thereafter directed through the channel and the mouthpiece in response to a negative pressure applied to the said one end of the mouthpiece.

The inhalation device according to the present invention, further comprises a nebulizer for nebulizing medicant supplied through the supplying means from an inhaler bottle, said nebulizer including said through channel, and means for maintaining the surfaces of the envelope apart from each other.

In use of a preferred embodiment in accordance with the present invention, the nebulizer is connected to a pressurized inhaler bottle. A predetermined quantity of aerosol and medicant is discharged from the bottle and directed into the envelope. The medicant enters the envelope in a first direction, and upon the application of a negative pressure to the mouthpiece by the patient, the contents of the envelope are delivered in a second direction, which is opposite to the inflating direction, to the patient's pulmonary tissue. By first filling the envelope with the aerosolized medicant, and then withdrawing the contents of the envelope into the patient's respiratory system, controlled inhalation of the medicant results, improving the rate and distribution of the medication in the respiratory tracts.

A device in accordance with the present invention may also be used as an incentive spirometer. Specifically, by inspiring through the mouthpiece, it is possible to sequentially deflate and inflate the envelope. The means for maintaining the interior surfaces of the envelope apart will prevent occlusion of the envelope which would interfere with a total evacuation of the envelope. With this structure, a consistent volume of air may be inspired. The envelope is preferably detachably connected to a mouthpiece or nebulizer to permit different sized envelopes to be used to correspond accurately to the volume capacity of a patient.

A device in accordance with the present invention may have an auditory signalling device for warning when a too rapid flow rate has been attained. The signaling device will therefore permit the patient to control the rate of inhalation.

There may be a series resistor means in the mouthpiece to produce a negative intrathoracic pressure which inhibits a rapid inhalation. Therefore, a controlled slow inhalation is made possible.

The patient can exhale back into the envelope and re-inspire over several breaths to aid in depositing aerosolized medicant which has not settled in the lung from previous breaths. This increases the effective dose of the medicant.

For a better understanding of the present invention and to show how it may be carried into effect, reference will now be made, by way of example, to the accompanying drawings, in which:

Figure 1 is an overall view of an inhaler device in accordance with a preferred embodiment;

Figure 2 is an exploded view of an inhaler apparatus as in a preferred embodiment of the invention;

Figure 3 is a longitudinal cross sectional view of a nebulizer suitable for use in the preferred embodiment of Figure 1;

Figure 4 is a top view, partially in cross section, of a preferred mouthpiece for use in an incentive spirometer;

Figure 5 is a top view of yet another embodiment of an incentive spirometer whereby a reed device is placed in the mouthpiece for indicating the flow rate therethrough;

Figure 6 is a side view, partially in cross section, ʰowing the use of this invention in connection with a relatively stiff foam rubber spring for the air bag;

Figure 7 is a side view, partially in cross section, showing another use of this invention with a conical spring stays;

Figure 8 is a side view similar to Figure 1, partially in section, utilizing plastic spring fingers inside the air bag;

Figure 9 is a side view, partially in section, showing the air bag in the form of a plastic dip molded bellow;

Figure 10 is a view partially in cross-section, showing the air bag with a compression spring; and

Figure 11 is a view partially in cross section of the invention, utilizing a gravity operated air bag.

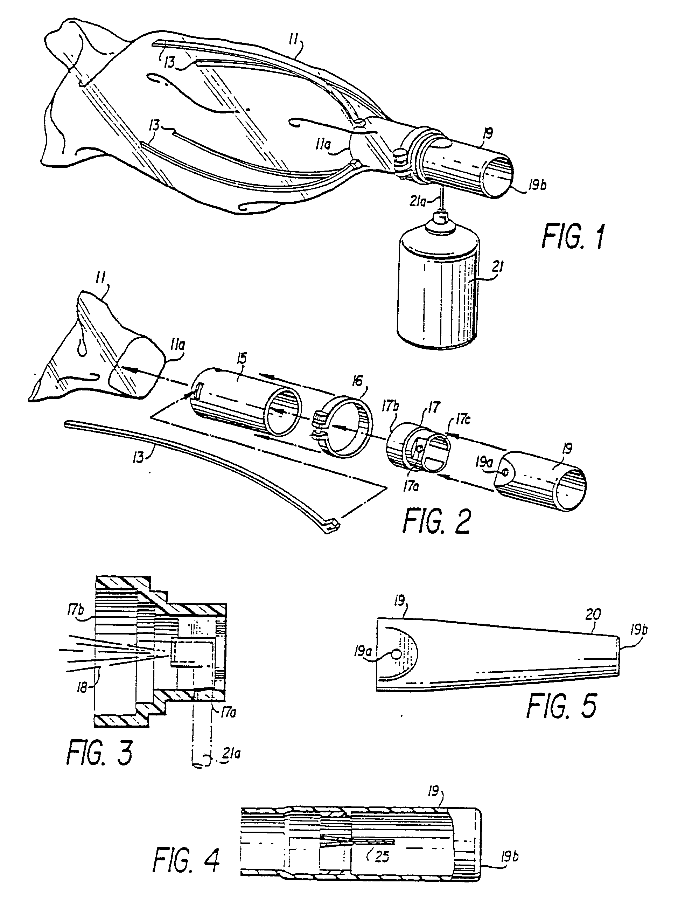

Figure 1 is a perspective view of an inhaler device in accordance with a preferred embodiment of the invention. For convenience it is shown in an inverted position and it should be understood that for most medicants it would be used with the bottle 21 extending upwardly. A plastic envelope 11 is provided having a plurality of interior surfaces which confine the medicant to be inhaled by a patient. The plastic envelope 11 has, within its interior a means for maintaining the surfaces apart which preferably comprise a plurality of resilient plastic stays 13 extending the length of the interior surfaces of the plastic envelope 11. The four plastic stays 13 shown in Figure 1 maintain the interior surfaces apart and after collapse of the bag, as a result of a negative pressure being applied to the outlet 11a thereof, the stays will force the interior surfaces of the plastic envelope 11 apart. When used as a spirometer, the stays prevents occlusion by virtue of air becoming trapped in the plastic envelope due to the interior surfaces contacting each other.

The inhaler device is used in connection with an inhaler bottle 21 which introduces a micronized medicant into the envelope 11. Once the envelope has received a charge of medicant from the inhaler bottle 21, the patient applies a negative pressure through his lips to the free end 91b of the mouth piece 19. The contents of the envelope 11 are thus drawn into the oral cavity of the patient.

Figure 2 shows an exploded view of the device of Figure 1. A plastic ring 15 is provided for mounting the four plastic stays 13 and the plastic envelope 11. The ring holds open an aperture at one end of the envelope whereby the medicant may be received and later discharged therethrough.

A nebulizer 17 is shown in association with the plastic ring 15, in Figure 3; the nebulizer communicates in a sealing fashion with the plastic ring 15. The inhaler bottle 21 and nebulizer 17, may be of a type available from Breon Company, sold as Bronchometer (TM) No. 1740 as part no. 7-1040-01. The nebulizer 17 may have an outlet 17b which has an outside diameter of a size to define an interference fit with the interior diameter of the plastic ring 15. This ring 15 is attached to the plastic envelope 1 either by applying tape, or by a small clamp 16 which may be easily removed by the patient using the device.

The nebulizer 17 is utilized by those skilled in the art to provide an aerosol mist 18 in response to the ejection through an opening 17a of a stream of aerosol propellent containing the desired medicant. The nebulizer 17 interfaces with a pressurized inhaler bottle 21 so that pressure applied to the nebulizer will discharge the micronized medicant through outlet 17b in a vapor stream. A through channel 17c permits the medicant in the envelope to turn and pass through to the mouthpiece 19.

Mouthpiece 19 has an inside diameter which fits snugly over with the outside diameter of the nebulizer 17. Opening 19a of the mouthpiece aligns with the opening 17a of the nebulizer. The stems of an inhaler bottle 21 may therefore protrude through openings 19a and 17a to contact a shoulder located within the nebulizer. By displacing the stem 21a longitudinally with respect to the inhaler bottle 21, a predetermined dosage of medicant is released. The nebulizer 17 directs the medicant into plastic envelope 11 which mixes the then micronized medicant with air therein.

To use the device shown in Figure 1, the patient places the free end of the mouthpiece 19b in his mouth. A quantity of medicant is discharged by moving the inhaler bottle 21 relative to the nebulizer 17. A predetermined dosage of aerosolized medicant is thereafter deposited within plastic envelope 11. When the patient applies a negative pressure to the free end of the mouthpiece 19b, the medicant stored within the plastic envelope 11 is drawn through the opening 17c in the nebulizer towards the mouthpiece 19 and into the repiratory system of the patient. Thus, with the device of Figure 1, it is possible to administer pulmonary medication in a two-step process. A patient, even small children, can first discharge the aerosol medicant into the plastic envelope 11, and thereafter breathe in the medicant to achieve a uniform dosage. By first directing the medicant into envelope 11, the coarser spray from the nebulizer is dispersed in the envelope reducing any impaction on the tongue of micronized medicant which would occur from the coarser spray of the nebulizer when introduced directly to the oral cavity. In addition the patient can exhale back into the plastic envelope and re-inspire once again over several breaths to aid in depositing the aerolized medicant which has not settled in previous breaths. This increases the effective dose of the medicant.

Figure 4 illustrates yet another embodiment of an incentive spirometer in accordance with the invention having an audio enunciator 25 for indicating when the flow rate through the mouthpiece is too rapid. When used as an incentive spirometer, the patient may visually and tactilely observe the inflation of the plastic envelope during breathing exercises. With the audio enunciator, which may be a simple reed whistle responding to an excessive flow rate, the patient is made aware by yet another means of a breathing performance as it relates to the air flow rate out of the envelope.

Figure 5 is yet another embodiment of a spirometer in accordance with the invention wherein the mouthpiece contains a restriction 20 which limits the air flow into and out of the spirometer. By including such a restriction device, the patient's breathing rate may be controlled so that a predetermined flow rate may be established in the incentive spirometer. Those skilled in the art will recognize therapeutic and prophylactic advantages resulting from such control.

Thus, with the device shown in Figure 1, it is possible to both administer pulmonary medication and conduct respiratory exercises in conjunction with the administration of pulmonary medication. The device provides for a long, slow inspiration period when distributing medication to the occluded airways, as well as providing the low and slow inspiration period during respiratory exercises which are desirable in treating postoperative atelectasis and clearing small passageways of the lungs.

A further variation on the embodiment of Figure 1 can include a mask connected to the mouthpiece which will facilitate the use of the device with children. The mask will snuggly fit over the face of the children and breathing into the envelope facilitated.

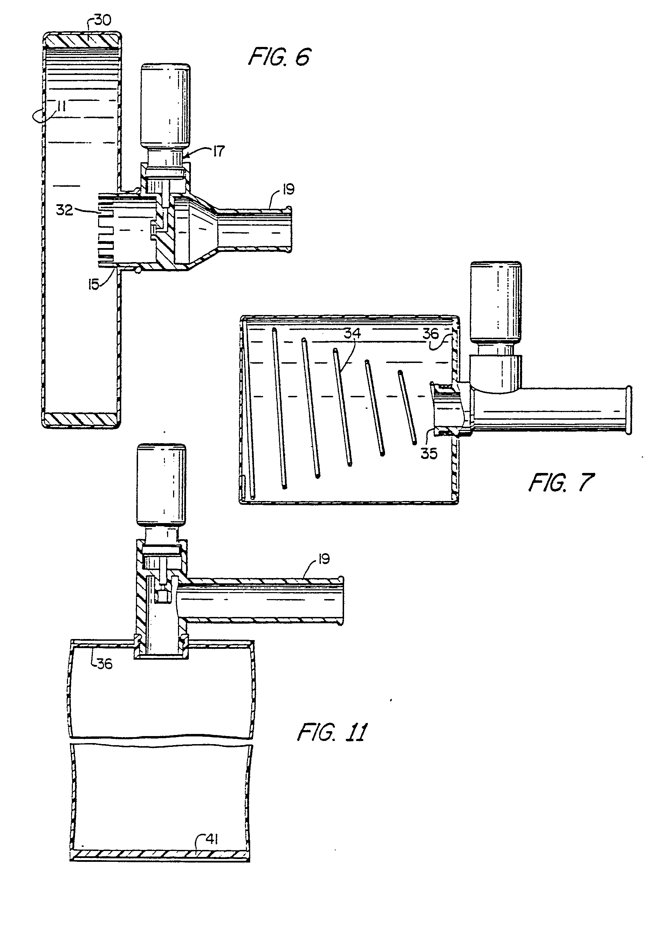

It is contemplated that the air bag 11 may be designed in several different forms as shown in Figures 6—11. These figures show the nebulizer 17 in an upright position. In Figure 6 the envelope 11 is in the form of a balloon bag which may be dip molded and shaped with an opening to define a stretch fit around tube 15. This tube has slots 32 on its inner end which upon inhalation, prevent closing off of the ends of the breathing tube by the collapsing bag. A relatively stiff foam rubber spring 30 is used to maintain the bag in its radially outwardly stretched position. Upon inhalation the bag can collapse to a limited extent, moving both radially inwardly toward the sleeve 15 and axially inwardly toward the slots 32.

The nebulizer 17 of this embodiment acts in the same manner as the nebulizer previously described. Similarly there is an open passage way 17c to permit air to return from the air envelope to the mouthpiece 19. The nebulizer in Figures 7—10 are similar and will not be separately described in detail. However, it should be noted that in all cases the outlet from the medicant supply faces towards the air bag so that the medicant is first deposited into the bag and mixed with air and then, upon subsequent suction inhalation by the patient, the aerosolized mixture then moves upstream toward the mouthpiece.

In Figure 7 the bag 11, which may be of condom type material, is held in its extended position by a conical spring spray device 34 which, when it is in its collapsed position, will form a substantially flat spiral concentrically about end 35 of the breathing tube and against end wall 36 of the device.

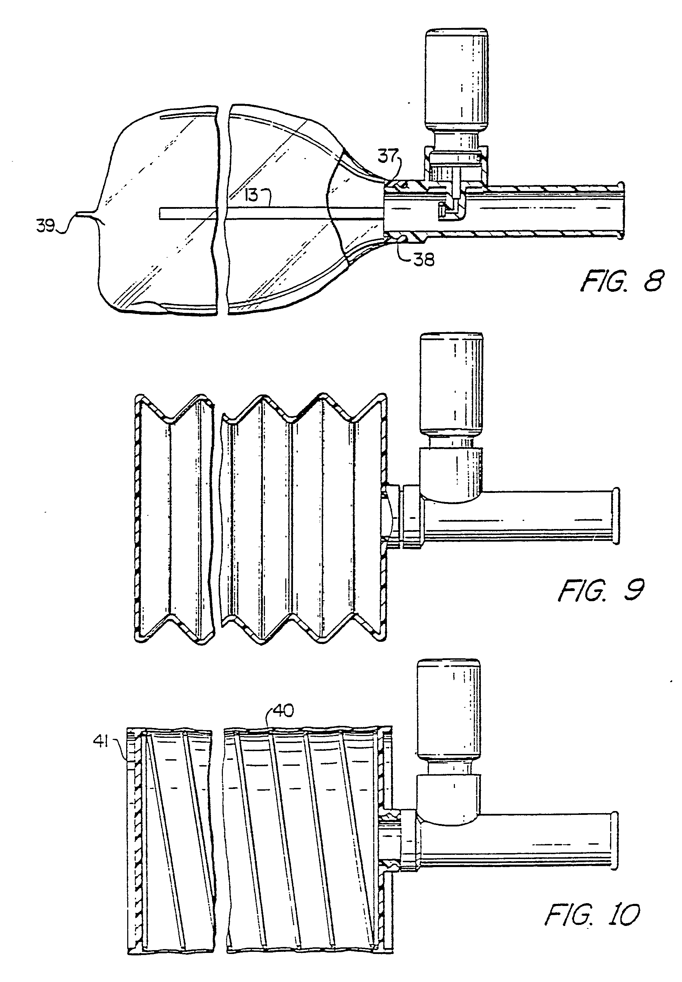

The inhalation device of Figure 8 is similar to that of Figure 1 but with a film bag which is held in place by a ring 37 which fits over a detent ring 38 on the end of the breathing tube. This bag can be fastened with a heat sealed distal end 39 and is kept open by plastic leaf springs 13 in the same manner and in the embodiment of Figure 1.

In the embodiment of Figure 9 the air bag is formed in the shape of a bellows which can collapse to a substantially flat position when not in use. This bellows can be formed of relatively thin polyethylene material such as 0,125 to 0,250 mm (.005 to .010 mil) thickness material.

The device of Figure 10 is similar to that of Figure 9 but with a compression spring 40 which is helically wound so as to radially outwardly support the walls of the bag which may be of relatively thin plastic material. The coil can, for example, comprise 6 coils of 0,762 mm (.03 mil) diameter music wire or a plastic material which is comparably resilient and light in weight. In both Figures 9 and 10 the bellows can be held to the breathing tube by a detent similar to that of Figure 8. The end cap 41 can be heat sealed to the plastic material used for the side wall. The materials would be selected so that the bag could compress axially due to breathing by a user.

In Figure 11 no positive spring pressure is necessary to maintain the bag in its open position because it is vertically arranged so as to be forced open by the effect of gravity with the breathing tube having a 90° bend in it. Thus as the mouthpiece 19 is held in a patient's mouth the medicant bottle of the nebulizer 17 would normally face upwardly as shown in Figure 11 while the end cap 41 hangs downwardly to hold the air bag open. The end cap is preferably of plastic material and of sufficient weight so as to hold the bag in its open position when no inhaling force is being applied but to piece the bag to partially collapse when the patient draws inwardly on the mouthpiece. The bag would preferably be made of a plastic film material which is heat sealed both to the end cap 41 and the opposite wall 36. Because of the 90° bend in the breathing tube, the medicant bottle is positioned so as to spray its contents directly into the air bag as in the previous discussion, although it in this case the path of the discharging medicant is, for a portion of its travel, perpendicular to the air flow path rather than being directly opposite to it as previously discussed. In all cases the operation is the same, to permit mixture of aerosolized medicant with the air in the bag prior to inspirational breathing by the patient.

## Claims

1. An inhalation device for administering pulmonary medication, comprising a mouthpiece (19) for delivering a micronized medicant, the mouthpiece (19) having one end (19b) for communication with an oral cavity, the mouthpiece (19) communicating with a through channel (17c) which has an inlet (17a) directed away from the said one end (19b) of the mouthpiece (19), the outlet (17b) of the through channel (17c) communicating with an open end (11a) of an inflatable envelope (11) having surfaces for confining a micronized medicant, the device further comprising means (21a) for supplying a predetermined amount of medicant to the through channel inlet (17a), so that, in use, a quantity of medicant enters the envelope (11) and is thereafter directed through the channel (17c) and the mouthpiece (19) in response to a negative pressure applied to the said one end (19b) of the mouthpiece (19), characterized in that the inhalation device further comprises a nebulizer (17) for nebulizing medicant supplied through the supplying means (21a) from an inhaler bottle (21), said nebulizer including said through channel (17c) and in that means is provided for maintaining the surfaces of the envelope (11) apart from each other.

2. An inhalation device as claimed in claim 1, characterized in that the envelope (11) is detachable.

3. An inhalation device as claimed in claim 1 or 2, characterized in that the means for maintaining the surfaces of the envelope (11) apart from each other comprises a plurality of spaced apart flexible stays (13) extending into the envelope (11).

4. An inhalation device as claimed in any one of the preceding claims, characterized in that means is provided for administering a predetermined amount of medicant into the channel (17c).

5. An inhalation device as claimed in any one of the preceding claims, characterized in that there is a restriction (20) in the mouthpiece (19) for limiting the flow rate of air through the mouthpiece (19).

6. An inhalation device as claimed in any one of the preceding claims, characterized in that audible warning means (25) is provided to indicate when the flow rate through the mouthpiece (19) exceeds a predetermined amount.

7. An inhalation device as claimed in claim 9, characterized in that the audible warning device (25) is a vibratory reed disposed within the mouthpiece (19).

## Revendications

1. Dispositif d'inhalation pour administration d'un médicament pulmonaire, comprenant une embouchure (19) destinée à délivrer un médicament micronisé, l'embouchure (19) ayant une première extrémité (19b) destinée à communiquer avec une cavité buccale, l'embouchure (19) communiquant avec un canal traversant (17c) qui présente une entrée (17a) tournée dans une direction s'éloignant de ladite extrémité (19b) de l'embouchure (19), la sortie (17b) du canal traversant (17c) communiquant avec une extrémité ouverte (11a) d'une enveloppe gonflable (11) présentant des surfaces destinées à retenir un médicament micronisé, le dispositif comprenant en outre des moyens (21a) destinés à fournir une quantité prédéterminée de médicament au canal d'entrée traversant (17a) de manière que, lors de l'utilisation, une quantité de médicament entre dans l'enveloppe (11) et soit ensuite dirigée dans le canal (17c) et dans l'embouchure (19) en réponse à l'application d'une pression négative à ladite première extrémité (19b) de l'embouchure (19), caractérisé en ce que le dispositif d'inhalation comprend en outre un nébuliseur (17) destiné à nébuliser un médicament fourni par les moyens (21a) de fourniture, à partir d'une bouteille (21) d'inhalateur, ledit nébuliseur comprenant ledit canal traversant (17c), et en ce que des moyens sont prévus pour maintenir mutuellement écartées les surfaces de l'enveloppe (11).

2. Dispositif d'inhalation selon la revendication 1, caractérisé en ce que l'enveloppe (11) est amovible.

3. Dispositif d'inhalation selon la revendication 1 ou 2, caractérisé en ce que les moyens destinés à maintenir mutuellement écartées les surfaces de l'enveloppe (11) comprennent plusieurs éléments espacées flexibles (13) d'appui pénétrant dans l'enveloppe (11).

4. Dispositif d'inhalation selon l'une quelconque des revendications précédentes, caractérisé en ce que des moyens sont prévus pour administrer une quantité prédéterminée de médicament vers l'intérieur du canal (17c).

5. Dispositif d'inhalation selon l'une quelconque des revendications précédentes, caractérisé en ce que l'embouchure (19) comporte un étranglement (20) destiné à limiter le débit d'écoulement d'air à travers l'embouchure (19).

6. Dispositif d'inhalation selon l'une quelconque des revendications précédentes, caractérisé en ce qu'il est prévu des moyens d'avertissement sonores (25) destinés à indiquer que le débit d'écoulement à travers l'embouchure (19) dépasse une valeur prédéterminée.

7. Dispositif d'inhalation selon la revendication 9, caractérisé en ce que le dispositif d'avertissement sonore (25) est une lame vibrante disposée à l'intérieur de l'embouchure (19).

**Patentansprüche**

1. Atemgerät zur medikamentösen Behandlung der Lunge, bestehend aus einem Mundstück (19) zur Abgabe eines feinstteiligen Arzneimittels, dessen ein Ende (19b) zur Verbindung mit der Mundhöhle dient und das mit einem Durchgangskanal (17c) verbunden ist, der einen von diesem Ende (19b) des Mundstückes (19) weggerichteten Einlaß (17a) aufweist und dessen Auslaß (17b) mit dem offenen Ende (11a) einer aufblasbaren Hülle (11) mit Flächen zur Umschließung des feinstteiligen Arzneimittels verbunden ist, und aus einer Einrichtung (21a) zur Zufuhr einer vorbestimmten Menge Arzneimittel zum Einlaß (17a) des Durchgangskanales (17c), so daß bei Gebrauch eine Menge Arzneimittel in die Hülle (11) eintritt und danach in Abhängigkeit von einem auf das genannte Ende (19b) des Mundstückes (19) ausgeübten Unterdruck durch den Kanal (17c) sowie das Mundstück (19) geleitet wird, dadurch gekennzeichnet, daß das Atemgerät weiters einen Zerstäuber (17) zum Zerstäuben des durch die Zufuhreinrichtung (21a) von einer Atemflasche (21) zugeführten Arzneimittels aufweist, wobei der Zerstäuber den Durchgangskanal (17c) aufweist, und daß eine Einrichtung zum gegenseitigen Auseinanderhalten der Flächen der Hülle (11) vorgesehen ist.

2. Atemgerät nach Anspruch 1, dadurch gekennzeichnet, daß die Hülle (11) abnehmbar ist.

3. Atemgerät nach Anspruch 1 oder 2, dadurch gekennzeichnet, daß die Einrichtung zum gegenseitigen Auseinanderhalten der Flächen der Hülle (11) aus mehreren im Abstand angeordneten biegsamen Spreizen (13) besteht, die sich in die Hülle (11) erstrecken.

4. Atemgerät nach den Ansprüchen 1 bis 3, dadurch gekennzeichnet, daß eine Einrichtung zur Abgabe einer vorbestimmten Menge Arzneimittel in den Kanal (17c) vorgesehen ist.

5. Atemgerät nach den Ansprüchen 1 bis 4, dadurch gekennzeichnet, daß im Mundstück (19) eine Verengung (20) zur Begrenzung der Durchflußmenge der Luft durch das Mundstück (19) vorgesehen ist.

6. Atemgerät nach den Ansprüchen 1 bis 5, gekennzeichnet durch eine hörbare Warneinrichtung (25) zur Anzeige, wenn die Durchflußmenge durch das Mundstück (19) einen vorbestimmten Betrag überschreitet.

7. Atemgerät nach Anspruch 6, dadurch gekennzeichnet, daß die hörbare Warneinrichtung (25) ein im Mundstück (19) angeordnetes Vibrationsblatt ist.

FIG. 1

FIG. 2

FIG. 3

FIG. 5

FIG. 4

**FIG. 6**

30

11

17

32

19

15

**FIG. 7**

34    36

35

**FIG. 11**

19

36

41

2

0 050 654

FIG. 8

FIG. 9

FIG. 10

3